(19) Europäisches Patentamt
European Patent Office
Office européen des brevets

(11) **EP 1 861 000 B1**

(12) **EUROPEAN PATENT SPECIFICATION**

(45) Date of publication and mention
of the grant of the patent:
**02.12.2009 Bulletin 2009/49**

(51) Int Cl.:
***A61B 5/026*** (2006.01)

(21) Application number: **06711029.6**

(22) Date of filing: **06.03.2006**

(86) International application number:
**PCT/IB2006/050693**

(87) International publication number:
**WO 2006/097866 (21.09.2006 Gazette 2006/38)**

(54) **METHOD AND DEVICE FOR DETERMINING THE PERFUSION OF BLOOD IN A BODY MEMBER**

VERFAHREN UND VORRICHTUNG ZUR BESTIMMUNG DER DURCHBLUTUNG IN EINEM KÖRPERELEMENT

PROCEDE ET DISPOSITIF PERMETTANT DE DETERMINER LA PERFUSION DE SANG DANS UN MEMBRE DU CORPS

(84) Designated Contracting States:
**AT BE BG CH CY CZ DE DK EE ES FI FR GB GR HU IE IS IT LI LT LU LV MC NL PL PT RO SE SI SK TR**

(30) Priority: **14.03.2005 EP 05101963**

(43) Date of publication of application:
**05.12.2007 Bulletin 2007/49**

(73) Proprietors:
• **Philips Intellectual Property & Standards GmbH 20099 Hamburg (DE)**
Designated Contracting States:
**DE**
• **Koninklijke Philips Electronics N.V. 5621 BA Eindhoven (NL)**
Designated Contracting States:
**AT BE BG CH LI CY CZ DK EE ES FI FR GB GR HU IE IS IT LT LU LV MC NL PL PT RO SE SI SK TR**

(72) Inventors:
• **NEUMANN, Rolf**
**Weisshausstr. 2,**
**52066 Aachen (DE)**
• **NOLLER, Friedemann**
**Weisshausstr. 2,**
**52066 Aachen (DE)**

(74) Representative: **Schouten, Marcus Maria**
**Philips**
**Intellectual Property & Standards**
**P.O. Box 220**
**5600 AE Eindhoven (NL)**

(56) References cited:
**EP-A- 0 807 402      US-A1- 2003 120 156**

**Description**

The invention relates to a method and device for determining the perfusion of blood in a body member. Furthermore, the invention relates to a computer program for determining the perfusion of blood in a body member.

As is known, in the field of patient monitoring, special patient data such as, for example, perfusion, oxygen saturation of the arterial blood ($SpO_2$ value), ECG curves and the like are important for the evaluation of the condition of a patient. Information as regards the perfusion can preferably be derived based on measuring values resulting from the measurement of the oxygen saturation of the arterial blood.

The measurement of the arterial oxygen saturation of the blood is usually performed continuously in a non-invasive manner by means of a photometric measuring process, that is, the so-called pulse oximetry. A peripheral part of the body, usually a finger, is then irradiated by means of a sensor. The sensor usually comprises two light sources for the emission of light and a corresponding photodetector for the measurement of the light absorption.

Pulse oximetry is based on two principles. On the one hand, the oxygen-enriched hemoglobin (oxyhemoglobin) and the oxygen-reduced hemoglobin (desoxyhemoglobin) differ as regards their ability to absorb red and infrared light (spectrophotometry) and, on the other hand, the amount of arterial blood in the tissue changes, and hence also the absorption of light by this blood, during the pulse (plethysmography). A pulsoximeter determines the $SpO_2$ value by emitting red and infrared light and by measuring the variations of the light absorption during the pulse cycle.

US 4,867,165 disclose a method for determining the perfusion, whereby the perfusion can be quantified. To this end, a so-called perfusion index is determined from measuring values of the pulse oximetry process by means of an algorithm. It has been found to be a drawback of this method, however, that depending on the patient conditions, in some cases the actual patient perfusion condition does not correlate with the calculated perfusion index value.

A method according to the preamble of claim 1 is known from EP 0 807 402.

It is an object of the present invention to provide a technique to ensure that the actual patient perfusion condition does correlate with the calculated perfusion index value over a broad variety of patient conditions.

This object is achieved according to the invention by a method of determining the perfusion of blood in a body member, the method comprising the steps of: producing electrical measuring signals from a non-invasive photometric measuring process, normalizing the electrical measuring signals and determining a perfusion index from the normalized signals, using an energy value of at least one signal pulse.

The object of the present invention is also achieved by a device for determining the perfusion of blood in a body member, comprising a signal component adapted for producing electrical measuring signals from a non-invasive photometric measuring process, and a determining component adapted for normalizing the electrical measuring signals and for determining a perfusion index from the electrical measuring signals, using an energy value of at least one signal pulse.

The object of the present invention is also achieved by a computer program for determining the perfusion of blood in a body member, comprising computer program instructions to determine a perfusion index from normalized electrical measuring signals from a non-invasive photometric measuring process, using an energy value of at least one signal pulse, when the computer program is executed in a computer. The technical effects necessary according to the invention can thus be realized on the basis of the instructions of the computer program in accordance with the invention. Such a computer program can be stored on a carrier or it can be available over the internet or another computer network. Prior to executing, the computer program is loaded into a computer by reading the computer program from the carrier, for example by means of a CD-ROM player, or from the internet, and storing it in the memory of the computer. The computer includes inter alia a central processor unit (CPU), a bus system, memory means, e.g. RAM or ROM etc., and input/output units.

A basic idea of the present invention is to determine a perfusion index value based on an energy value of at least one signal pulse, i.e. independently of the specific shape of the signal pulse. To this end, a non-invasive photometric measuring process is utilized, the measuring process preferably being adapted for determining the arterial oxygen saturation of blood. The electrical measuring signals are normalized before being processed. With the normalizing step a perfusion index value can be obtained which is nearly independent of measuring conditions such as light source, tissue composition, type of sensor, etc.

The pulse shapes of the electrical measuring signals may be different, depending on the patient's condition. For example sharp peaks or flat pulses may be an indication of a patient's disease. If the pulses show different shapes, the use of prior art methods to calculate a perfusion index would result in significantly different values, since those methods rely mostly on the pulse amplitude. In contrast to those prior art methods, the present technique provides perfusion index values taking into account an energy value, preferably the energy content value of the signal pulse. The energy content value corresponds to an area under the pulse curve, thus leading to a perfusion index value that is independent of the specific pulse shape. As a result, the actual patient perfusion condition does correlate correctly with the calculated perfusion index value over a broad variety of patient conditions.

Since the perfusion index value can be obtained by using the signals from the pulse oximeter measurement,

EP 1 861 000 B1

no additional sensors or the like are needed. The optical signals can be obtained using at least one operating wavelength.

These and other aspects of the invention will be further elaborated on the basis of the following embodiments, which are defined in the dependent claims.

According to a preferred embodiment of the invention, the normalizing of the electrical measuring signals is carried out with respect to the direct current level of the electrical measuring signals.

According to another preferred embodiment of the invention, the signal curve from the end of the diastole of a heart beat to the end of the diastole of the next heart beat is used for determining the energy value. This enables a very easy approach to obtain an energy value corresponding to the area under the signal curve.

In another preferred embodiment of the invention, the signal curve is analysed and only a part of the pulse is used for determining the energy value, e.g. the part of the pulse corresponding to the systole or the diastole of the heartbeat.

In still another embodiment of the invention, the area of the pulse is estimated using a number of predefined geometric figures in order to determine an energy value, e.g. by using the area of a triangle that is defined by the minima and maxima of the pulse.

To obtain enhanced results in another embodiment of the invention, the impact of the blood saturation on the signals for wavelengths other than the isobestic wavelength is compensated for. In another preferred embodiment of the invention, the signals from the sensor are pre-filtered and/or artifact-reduced before the perfusion is determined.

These and other aspects of the invention will be described in detail hereinafter, by way of example, with reference to the following embodiments and the accompanying drawings; in which:

    Fig. 1 is a schematic picture of a measuring sensor, which is fitted on the tip of a finger,
    Fig. 2 is a schematic diagram showing tissue with arterial and venous blood flow,
    Fig. 3 is a diagram showing the light intensities at the photodiode from two LEDs with different wavelengths,
    Fig. 4 illustrates the absolute light intensity attenuation for two different wavelengths,
    Fig. 5 shows a pulse pattern with normal pulse shape,
    Fig. 6 shows a pulse pattern with abnormal pulse shape,
    Fig. 7 shows another pulse pattern with abnormal pulse shape,
    Fig. 8 shows a pulse pattern with an area related to a complete pulse,
    Fig. 9 shows a pulse pattern with an area related to the systole phase of the heartbeat,
    Fig. 10 shows a pulse pattern with a triangular estimate of the pulse area,
    Fig. 11 is a diagram showing the light absorption for hemoglobin and oxyhemoglobin,
    Fig. 12 illustrates the normalized and logarithmic light intensity attenuation for two different wavelengths,
    Fig. 13 is a schematic block diagram illustrating the device for determining the perfusion, and
    Fig. 14 is a schematic block diagram illustrating the device for determining the perfusion when use is made of filtering methods that eliminate or reduce signal artifacts.

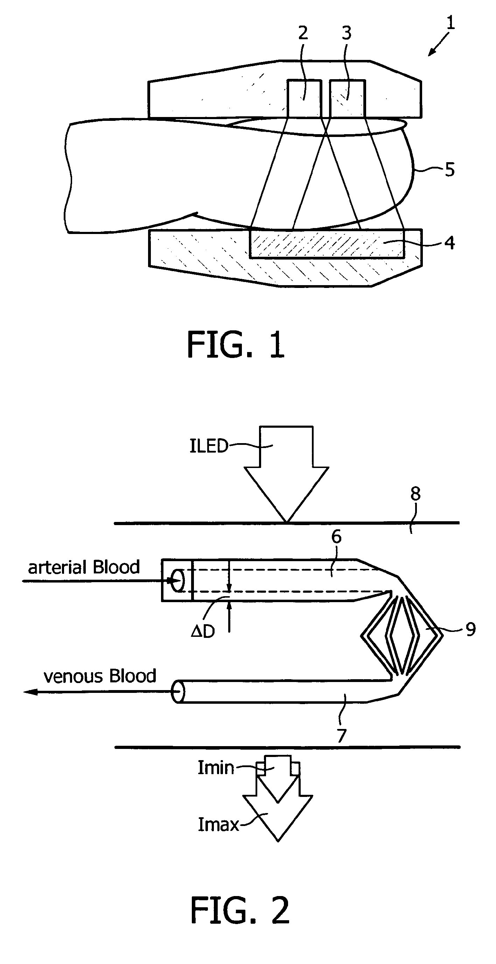

Pulse oximetry is a spectrophotometric method for the non-invasive determination of the arterial oxygen saturation of the blood. To this end, use is made of a measuring sensor 1 as shown in Fig. 1, which sensor 1 comprises two light-emitting diodes 2, 3 and a photoreceiver 4 and is fitted on the tip of a finger 5 of the patient to be monitored. The measuring sensor 1 can of course also be placed on another appropriate part of the patient's body.

The arteries 6 and veins 7 extending in the tissue 8 and the capillaries 9 present between the arteries 6 and veins 7 are shown in Fig. 2. The light-emitting diodes 2, 3 emit light of different wavelength, for example 650 nm and 940 nm, to the photoreceiver 4. Thus, light with the intensity $I_{LED}$ is transmitted through a part of the body, here through tissue 8, and is attenuated to a certain degree, which is dependent on the various tissue layers and the instantaneous volume of the artery 6. The photoreceiver 4 measures the intensity variation of the light, which is due to a variation in arterial blood volume and converts this information into a current signal. The pulsation of arterial blood causes a pulsating volume variation of the effective thickness $\Delta d$ of arteries 6 and arterioles, which correlates with the variation of the light intensity ($I_{max}$-$I_{min}$) on the receiver side. This effect is independent of the fact whether the sensor used is a so-called 'transmission sensor', as shown as an example in Fig. 1, or a so-called 'reflectance sensor', where the photoreceiver is on the same side of the tissue as the light-emitting diodes.

Since photometric measuring methods are well known, for the sake of simplicity the measuring method will not be shown or elaborated further.

In order to enable direct representation of the variations of the perfusion of a patient, an algorithm is used to determine the so-called perfusion index from the measuring values continuously produced by pulse oximetry. The monitoring of this perfusion index value and its trend, which gives a normalized plethysmogram, can be very useful in clinical situations. For example, a volume change of arterial blood can indicate a change in sympathicotonus.

A practical numerical value for the change of the pulsatile arterial blood flow gives the perfusion index:

$$\text{Perf } [\%] = f(I(t))_{\lambda_{iso}} \qquad (1)$$

Therein $\lambda_{iso}$ indicates that the light source emits at an isobestic wavelength ($\lambda$=810nm), which makes this definition independent of the actual blood saturation. I(t) indicates the actual light intensity.

A pulse oximeter normally comprises two LEDs 2, 3 with two different wavelengths $\lambda_1$ and $\lambda_2$ and does not use an isobestic wavelength. In this case the perfusion index Perf can be derived as a linear combination of both signals and the constants $k_1$ and $k_2$, which are functions of the different extinction coefficients:

$$\text{Perf } [\%] = k_1 \cdot f(I_1(t))_{\lambda_1} + k_2 \cdot f(I_2(t))_{\lambda_2} \qquad (2)$$

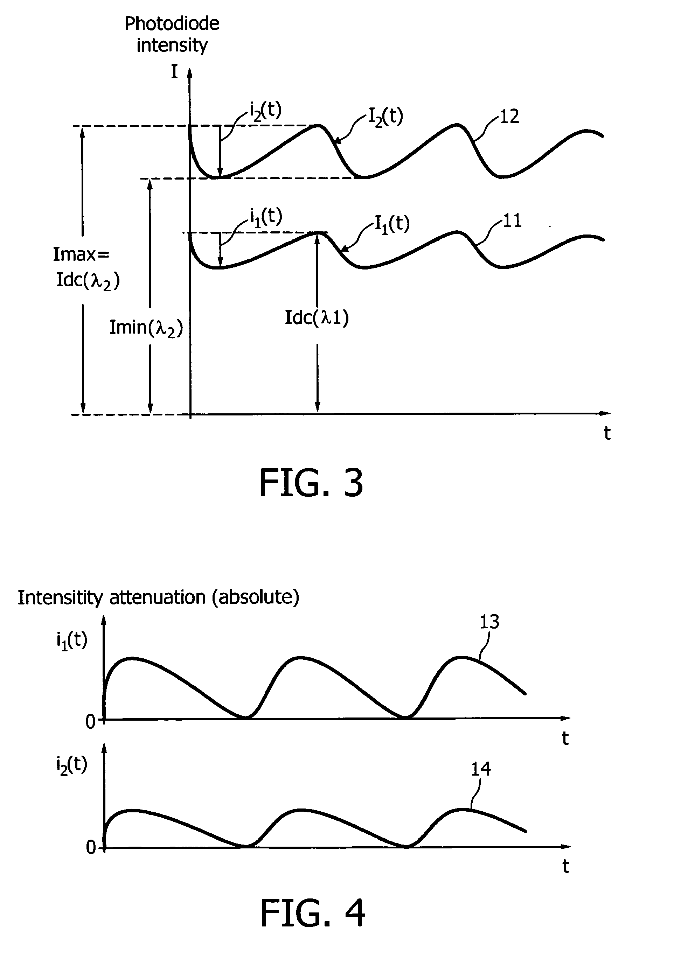

The maximum light intensity $I_{max}$ is equal to the non-pulsatile light intensity, see Fig. 3, and depends on the LED-intensity $I_0$, the absorption $E_n c_n$ and the thickness $d_n$ of all non-pulsatile components. $E_n$ indicates the effective molecular extinction coefficient of the n absorbers and $c_n$ indicates the effective molecular concentration of the n absorbers. In Fig. 3, a first pulse wave $I(\lambda_1)$ 11 and a second pulse wave $I(\lambda_2)$ 12 is shown.

$I_{max}$ is given by the Lambert-Beer law:

$$I_{max} = I_{dc} = I_0 \exp(-(E_n c_n d_n)) \qquad (3)$$

where $E_n$, $c_n$ and $d_n$ of the n absorbers are not explicitly known. $I_{dc}$ indicates the non-pulsatile, static component of the signal. The actual light intensity attenuation i(t) is given by:

$$i(t) = I_{dc} - I(t) \qquad (4),$$

the actual light intensity at a point in time I(t) can be expressed as:

$$I(t) = I_{dc} - i(t) = I_0 \exp[-(E_n c_n d_n)] \exp[-(E_{Hb} c_{Hb} + E_{HbO2} c_{HbO2}) \cdot \Delta d(t)] \qquad (5)$$

Using equation (4), the photodiode intensity signals can be converted to terms of light intensity attenuation, as shown in Fig. 4. Here the absolute intensity attenuations $i_1(t)$ 13 and $i_2(t)$ 14 are shown for the two wavelengths.

From the prior art it is known to estimate the perfusion from such a light intensity attenuation signal. The disadvantage of those approaches is that the absolute light level $I_0$ can be very different from case to case due to LED power variations and the variations in the transparency of the measurement site for different patients.

To get a meaningful numerical value that is independent of those variations, the resulting light levels have to be normalized in regard to those effects. The best value to use is $I_{dc}$, as this term is proportional to $I_0$ as well as to the unknown, patient dependent static attenuation $\exp(-(E_n C_n d_n))$. Using equation (3), it follows that:

$$I(t)/I_{dc} = 1 - i(t)/I_{dc} = \exp[-(E_{Hb} c_{Hb} + E_{HbO2} c_{HbO2}) \cdot \Delta d(t)] \qquad (6)$$

As the effective variation of arterial thickness $\Delta d(t)$ is caused by the arterial pulse, a term x(t) is derived that is directly proportional to $\Delta d(t)$

$$x(t) = -\ln(1 - i(t)/I_{dc}) = (E_{Hb} c_{Hb} + E_{HbO2} c_{HbO2}) \cdot \Delta d(t) \qquad (7)$$

Known methods to derive a normalized perfusion index value use the maximum value $x_{max}$ of each pulse, i.e. I(t) = $I_{min}$. Those methods provide a good relationship between changes in the calculated perfusion index and changes in the perfusion only for normal pulse shapes. But with pulse shapes different from normal, the perfusion index Perf is underestimated or overestimated. This is because not only the peak value of a pulse ($\Delta d_{max}$) is responsible for the amount of blood flow. It is any point in time of a pulse that contributes, to a certain amount, to the overall blood flow. Therefore, only a method to calculate the perfusion index Perf, which takes into account the pulse as a whole, can show superior performance in estimating the perfusion. Such a method is suggested by the present invention.

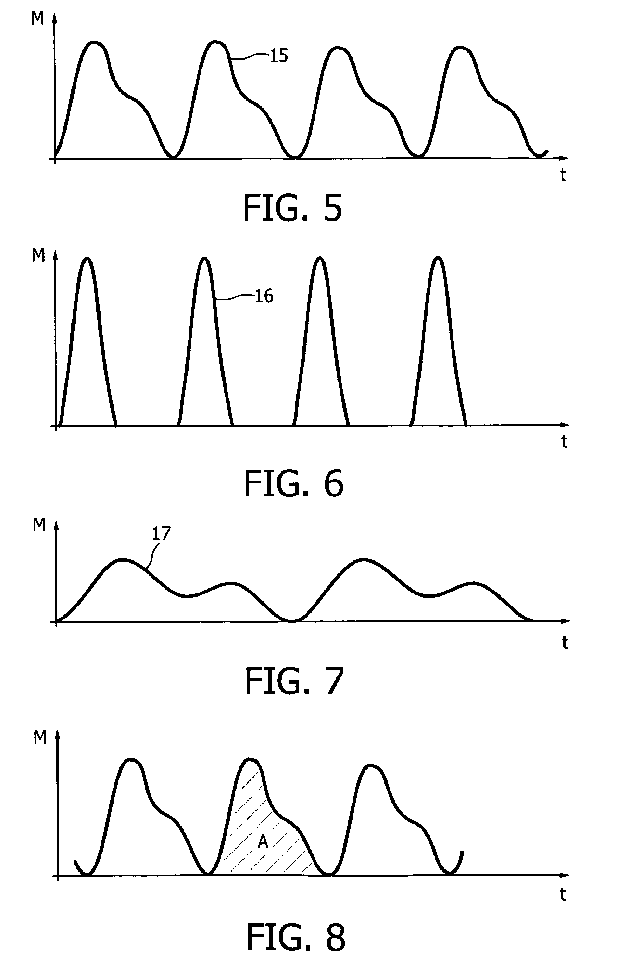

Fig. 5 shows a normal pulse shape 15, for which known methods would determine a correct perfusion index value. Fig. 6 shows a pulse shape 16 corresponding to an insufficiency of the aorta. Prior art perfusion index determination methods would provide too high Perf values. Fig. 7. shows a pulse shape 17 corresponding to a low peripheral resistance. Prior art perfusion index determination methods would provide too low Perf values. A correct perfusion index value for cases as shown in Figs. 6 and 7 as well as for other cases, can be determined using the present invention.

In a first embodiment of the invention, each point of the pulse is used with equal weight to calculate the overall contribution to blood flow of that pulse, resulting in a mean value x'. Using equation (7) this results in:

$$Perf \approx x' = \frac{A_{\log}}{T_{pulse}} = \frac{1}{T_{pulse}} \int\limits_{startofpulse}^{endofpulse} x(t)dt \qquad (8)$$

with $T_{pulse}$ = end of pulse - start of pulse. $A_{\log}$ represents the area under the logarithmic function x(t) as defined in equation (7) for one pulse. Preferably x' is determined for more than one pulse, i.e. over a longer period of time, in order to obtain a certain averaging effect.

In a variation of this method, a weighting function is used to express a non-linear relationship between $\Delta d(t)$ and the blood flow in the tissue. Preferably a quadratic relationship is used as follows:

$$y(t) = x^2(t) \qquad (9)$$

The resulting perfusion index value Perf is then calculated as:

$$Perf \approx y' = \sqrt{\frac{A_{sq}}{T_{pulse}}} = \sqrt{\frac{1}{T_{pulse}} \int\limits_{startofpulse}^{endofpulse} x^2(t)dt} \qquad (10)$$

$A_{sq}$ represents the area under the quadratic function y(t) as defined in equation (9) for one pulse. It is obvious that other functions can be used to model the relationship between the measured $\Delta d$ and the corresponding blood flow in the measured tissue.

In this embodiment, the area A under the signal curve (measure M of arterial width variation over time t) is used for a complete pulse, i.e. from the end of the diastole of a heart beat to the end of the diastole of the next heart beat, as illustrated in Fig. 8. This technique enables a close correlation of the calculated perfusion index with the changes of the actual perfusion and/or blood flow of the patient, independent of other factors like pulse shape or heart rhythm, see Figs. 5 to 7.

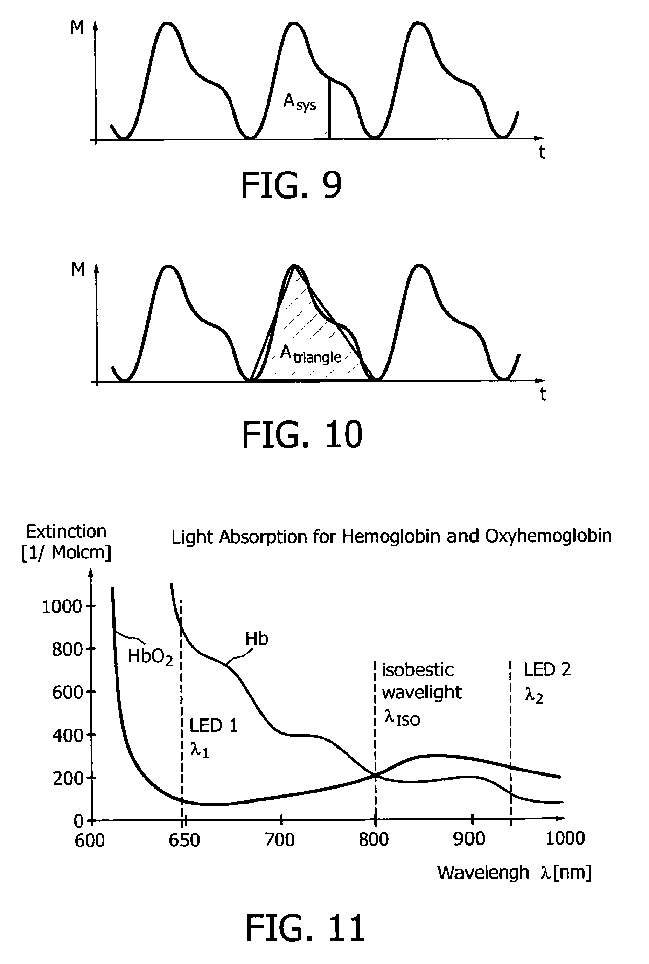

In a further embodiment of the present invention, not the complete pulse is used. Instead only the part of the pulse is used that corresponds to the systole of the heartbeat. With this approach the pulse has to be evaluated first as regards its properties to determine the start of systole, typically the time of maximum light intensity (minimal attenuation), and the end of systole, e.g. the peak of the pulse or the inflection point after the peak of the pulse, see Fig. 9. Here only the area $A_{sys}$ under the curve corresponding to the systole is used. In another embodiment (not shown), only the part of the pulse is used that corresponds to the diastole of the heartbeat. Both methods use properties of the signal shape and are preferably combined with amplitude/peak measurements.

In another embodiment of the invention, the whole pulse is used, as illustrated in Fig.8. However, the area of the pulse is estimated by using the area $A_{triangle}$ of the triangle that is defined by the minima and maxima of the pulse see Fig. 10. The advantage of this embodiment is an easier and faster determination of the relevant area.

Another embodiment of the invention uses a transformation of the measuring signal that results in energy related values for determining the perfusion index. E.g. a fourier transformation can be used for this purpose.

All embodiments have in common that they estimate the impact of $\Delta d$ on the blood flow. For the further discussion these estimates for the impact on the blood flow are generally called $\Delta D$ as the following can be applied to all embodiments described above.

The pulsatile arterial blood consists of two main absorbers hemoglobin (Hb) and oxyhemoglobin (HbO$_2$), which has different light absorption characteristics, and $E_{Hb}$ indicates the extinction of hemoglobin and $E_{HbO2}$ indicates the extinction of oxyhemoglobin. The extinction coefficients for hemoglobin Hb and oxyhemoglobin HbO$_2$ depend on the wavelength of the irradiated light. The pulse oximeter used as a <u>source of the light absorption values</u> shown in Fig. 11, emits at $\lambda_1$=650 nm and $\lambda_2$=940nm.

All methods described above can be applied with the use of only one wavelength. For the single wavelength approach the optimal wavelength is the isobestic wavelength $\lambda_{iso}$, i.e. the wavelength at which the hemoglobin in blood has the same extinction independent of whether it is loaded with oxygen (oxyhemoglobin) or not. For an isobestic wavelength ($E_{Hb}=E_{HbO2}$) the perfusion index is given by:

$$Perf = \frac{A_{\lambda iso}}{T_{pulse}} = E_{Hb\lambda iso}[Hb]\Delta D \qquad (11)$$

$A_{\lambda iso}$ represents the corresponding area under the curve that is obtained when the measurement is performed at the isobestic wavelength $\lambda_{iso}$. $E_{Hb\lambda iso}$ indicates the molecular extinction coefficient of hemoglobin at the isobestic wavelength $\lambda_{iso}$. Typically, a pulse oximeter is employed for determining the oxygen saturation S in blood, using two wavelengths that are different from $\lambda_{iso}$. The use of a wavelength other than $\lambda_{iso}$ causes a dependency of the light attenuation that is not related to the perfusion, but instead to the saturation. To overcome that problem, the methods described above are preferably enhanced by using the actual saturation value to compensate for that effect, thereby making the perfusion index Perf independent of oxygen saturation S.

There are two principle ways to carry out the compensation. Both approaches use the following definitions. The total hemoglobin concentration [Hb] is defined as:

$$[Hb] = c_{Hb} + c_{HbO2} \qquad (12)$$

The oxygen saturation S is defined as:

$$S = c_{HbO2}/[Hb] \qquad (13)$$

Using equations (12) and (13) to substitute the term ($E_{Hb}C_{Hb}+E_{HbO2}C_{HbO2}$) of equation (7) results in

$$(E_{Hb}c_{Hb}+E_{HbO2}c_{HbO2}) = (E_{Hb}(1-S) + E_{HbO2}S) [Hb] \qquad (14)$$

with $E_{Hb}$ and $E_{HbO2}$ being the corresponding extinction factors at the wavelength used.

In a first approach a correction factor is calculated based on the actual saturation value together with a compensation function that depends on the wavelength used. With the relationship given above, the correction function c (S) can be defined as:

$$c(S) = 1/(E_{Hb}(1-S) + E_{HbO2}S) \qquad (15)$$

With this correction function the perfusion index Perf is calculated as:

$$Perf = \frac{A_{\lambda used}}{T_{pulse}} \cdot \frac{1}{E_{Hb}(1-S) + E_{HbO2}S} = [Hb]\Delta D \qquad (16)$$

$A_{\lambda used}$ represents the corresponding area under the curve that is obtained when the measurement is performed at a wavelength $\lambda_{used}$ other than the isobestic wavelength $\lambda_{iso}$. However, this approach has the disadvantage that often there is a delay between the "actual" saturation S (e.g. as determined by the pulse oximeter) and the data used to calculate the perfusion index Perf.

The second approach is independent of delays between different processing stages as only one data set is used to calculate the perfusion index Perf. This data set comprises data from at least two wavelengths.

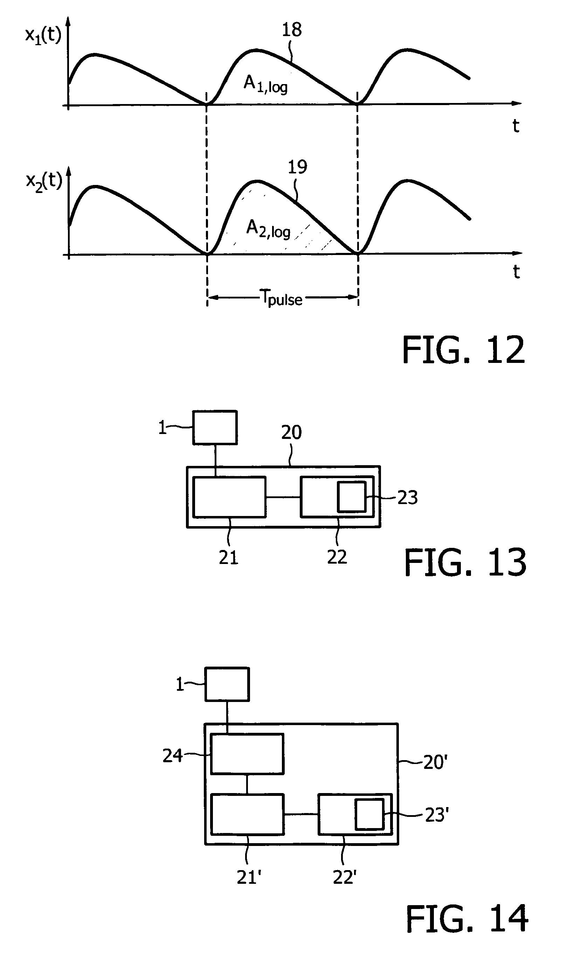

The data from each of the at least two wavelengths is processed according to equation (7), resulting in normalized and logarithmic light intensity attenuations 18, 19 for wavelengths $\lambda_1$ and $\lambda_2$ that are shown in Fig. 12. In the case of two different wavelengths $\lambda_1$ and $\lambda_2$, the saturation S in equation (14) can be eliminated and the product [Hb] $\Delta D$ can be expressed as a function of different extinction coefficients and the mean intensity attenuation values:

$$[Hb] \Delta D = k_1 \cdot x'_1 + k_2 \cdot x'_2 \qquad (17)$$

Accordingly, the perfusion index Perf results in:

$$Perf = k_1 \frac{A_{\lambda 1}}{T_{pulse}} + k_2 \frac{A_{\lambda 2}}{T_{pulse}} = [Hb]\Delta D \qquad (18)$$

with

$$k_1 = (E_{Hb} - E_{HbO2})\lambda_2 / (E_{Hb}\lambda_1 E_{HbO2}\lambda_2 - E_{Hb}\lambda_2 E_{HbO2}\lambda_1)$$

and

$$k_2 = (E_{HbO2} - E_{Hb})\lambda_1 / (E_{Hb}\lambda_1 E_{HbO2}\lambda_2 - E_{Hb}\lambda_2 E_{HbO2}\lambda_1).$$

For typical pulse oximeter signals the dc (direct current) component is much larger than the ac (alternating current) component, which allows to replace the logarithmic calculation in equation (7) by an approximation that needs less calculation power. If $I_{ac} \ll I_{dc}$, it follows from $\ln(1-x) = - (x + x^2/2 + x^3/3 + ..)$ that

$$x(t) = - \ln (1 - i(t)/I_{dc}) = (E_{Hb}c_{Hb} + E_{HbO2}c_{HbO2}) \cdot \Delta d(t) \cong i(t)/I_{dc} \qquad (19)$$

and so all previously described methods can also make use of this simplification, i.e. replacing the logarithmic calculation by the approximation presented in equation (19).

An example will be given as follows: If $\lambda_1 = 650nm$ and $\lambda_2 = 940nm$ and $\lambda_{iso} = 810nm$, the extinction coefficients are

$$E_{Hb}\lambda_1 = 820 \ 1/Molcm$$

$$E_{HbO2}\lambda_1 = 100 \ 1/Molcm$$

$$E_{Hb}\lambda_2 = 100 \ 1/Molcm$$

$$E_{HbO2}\lambda_2 = 260 \ 1/Molcm$$

$$E_{Hb}\lambda_{iso} = 200 \ 1/Molcm$$

and the perfusion index Perf is determined as:

$$Perf = 0,16 \cdot A_{1,\log} + 0,71 \cdot A_{2,\log} = \frac{0,16}{T_{pulse}} \int x_1(t)dt + \frac{0,71}{T_{pulse}} \int x_2(t)dt =$$

$$= \frac{0,16}{T_{pulse}} \int -\ln(1 - \frac{i_1(t)}{I_{1,DC}})dt + \frac{0,71}{T_{pulse}} \int -\ln(1 - \frac{i_2(t)}{I_{2,DC}})dt \qquad (20)$$

with

$A_{1,\log}$    area under the normalized logarithmic light intensity attenuation curve for wavelength $\lambda_1$,
$A_{2,\log}$    area under the normalized logarithmic light intensity attenuation curve for wavelength $\lambda_2$,
$i_1(t)$    light intensity attenuation signal for wavelength $\lambda_1$,
$i_2(t)$    light intensity attenuation signal for wavelength $\lambda_2$,
$I_{1,DC}$    DC value of the light intensity signal for wavelength $\lambda_1$, and
$I_{2,DC}$    DC value of the light intensity signal for wavelength $\lambda_2$.

All methods described in the invention can be applied to "raw" light intensity/ intensity-attenuation signals as well as to pre-filtered (artefact reduced) signals.

In pulse oximetry various different approaches are known to filter the light intensity signals to reduce or eliminate the effect of signal artefacts, especially motion artefacts. Examples of those filter approaches are: noise canceller, notch filters, adaptive filters, filters that operate in the frequency domain. Those methods are intended to filter out all signal components that are not related to the arterial pulsation. Many of those approaches result in pre-filtered (artefact reduced) intensity or intensity-attenuation signals (normalized or not) that can be used directly or indirectly (via transformation means) by the methods described in the invention to obtain an artefact-free or artefact-reduced perfusion index that is based on values that correspond to the energy of the arterial pulsation. If a transformation is used that conserves the energy content of the signal (e.g. Parseval's Theorem for Fourier Transform), the corresponding transformation value that represents the pulse energy can also directly be used to calculate the perfusion index. This could be done for example according to equation (18) by replacing the terms $A_{\lambda i}/T_{pulse}$ by the corresponding transformation value that represents the pulse energy for that wavelength $\lambda_i$.

In Fig. 13, a device 20 for determining the perfusion of blood in a body member is shown. The device 20 comprises a signal component 21 connectable to the measuring sensor 1. The signal component 21 produces electrical measuring signals from the photometric measuring process. Furthermore, the device 20 comprises a determining component 22 for determining the perfusion index from the electrical measuring signals according to one of the above-described methods. For this purpose, the determining component 22 is connected to the signal component 21 in order to provide a data transfer channel for the electrical measuring signals. The determining component 22 comprises a computer adapted to execute a computer program 23 according to the present invention. Preferably, the computer program 23 is adapted to determine a perfusion index value according to equation (18), when the computer program 23 is executed in the computer. In other words, a perfusion index value is calculated using normalized logarithmic light intensity attenuations that are integrated over at least a heart beat to obtain a value that represents an energy value of the pulse, independent of its specific shape.

In Fig. 14, another embodiment of the device 20' for determining an artefact-free or artefact-reduced perfusion of blood in a body member is shown. The device 20' comprises a signal component 21' connectable to the measuring sensor 1. The signal component 21' produces electrical measuring signals from the photometric measuring process, which are filtered/artifact-reduced by filter means 24. Furthermore, the device 20' comprises a determining component 22' for determining the perfusion index from the filtered/artifact-reduced signals according to one of the methods described above. For this purpose, the determining component 22' is connected to the filter means 24 in order to provide a data transfer channel for the filtered/artifact-reduced signals. The determining component 22' comprises a computer adapted to execute a computer program 23' according to the present invention. Preferably, the computer program 23' is adapted to determine a perfusion index value according to equation (18), when the computer program 23' is executed in the computer. In other words, a perfusion index value is calculated using normalized logarithmic light intensity attenuations that are pre-filtered and artifact-reduced before they are integrated over at least a heart beat to obtain a value that represents an energy value of the pulse, independent of its specific shape.

It will be evident to those skilled in the art that the invention is not limited to the details of the foregoing illustrative embodiments, and that the present invention may be embodied in other specific forms without departing from the spirit or essential attributes thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. It will furthermore be evident that the word "comprising" does not exclude other elements or steps, that the words "a" or "an" do not exclude a plurality, and that a single element, such as a computer system or another unit, may fulfil the functions of several means recited in the claims. Any reference signs in the claims shall not be construed as limiting the claim concerned.

REFERENCE LIST

| | |
|---|---|
| 1 | measuring sensor |
| 2 | LED |
| 3 | LED |
| 4 | photo receiver |
| 5 | finger |
| 6 | artery |
| 7 | vein |
| 8 | tissue |
| 9 | capillary |
| 10 | free |
| 11 | pulse wave resulting from one wavelength |
| 12 | pulse wave resulting from another wavelength |
| 13 | intensity attenuation of one wavelength |
| 14 | intensity attenuation of another wavelength |
| 15 | normal pulse shape |
| 16 | abnormal pulse shape |
| 17 | abnormal pulse shape |
| 18 | normalized intensity attenuation of one wavelength |
| 19 | normalized intensity attenuation of another wavelength |
| 20 | device for determining perfusion |
| 21 | signal component |
| 22 | determining component |
| 23 | computer program |
| 24 | filter means to provide pre-filtered/artefact-reduced signals |

**Claims**

1.   A method of determining the perfusion of blood in a body member (5), the method comprising the steps of producing electrical measuring signals from a non-invasive photometric measuring process, normalizing the electrical measuring signals and determining a perfusion index (Perf) from the normalized signals, **characterised in that** the perfusion index is determined from the normalised signals using an energy value (A) of at least one signal pulse.

**2.** The method as claimed in claim 1, wherein normalizing the electrical measuring signals comprises normalizing the electrical measuring signals with respect to the direct current level of the electrical measuring signals.

**3.** The method as claimed in claim 1, wherein the signal curve from the end of the diastole of a heart beat to the end of the diastole of the next heart beat is used for determining the energy value (A).

**4.** The method as claimed in claim 1, wherein the signal curve is analysed and only a part of the pulse is used for determining the energy value (A).

**5.** The method as claimed in claim 1, wherein the area of the pulse is estimated using a number of predefined geometric figures in order to determine an energy value (A).

**6.** The method as claimed in claim 1, wherein the impact of the blood saturation on the signals for wavelengths other than the isobestic wavelength is compensated for.

**7.** The method as claimed in claim 1, wherein the signals from the sensor are pre-filtered and/or artifact-reduced before the perfusion is determined.

**8.** A device (20) for determining the perfusion of blood in a body member (5), comprising a signal component (21) adapted for producing electrical measuring signals from a non-invasive photometric measuring process, and **characterised by** a determining component (22) adapted for normalizing the electrical measuring signals and for determining a perfusion index (Perf) from the normalized signals, using an energy value (A) of at least one signal pulse.

**9.** The device (20) as claimed in claim 8, wherein the signal component (21) is adapted for producing electrical measuring signals from a non-invasive photometric measuring process for determining the arterial oxygen saturation of the blood, the device (20) further comprising filter means (24) for reducing or eliminating the effect of signal artefacts, and wherein the determining component (22) is adapted for determining the perfusion index (Perf) from the pre-filtered and artifact-reduced signals.

**10.** A computer program (23) for determining the perfusion of blood in a body member (5), comprising computer program instructions to determine a perfusion index (Perf) from normalized electrical measuring signals from a non-invasive photometric measuring process, when the computer program (23) is executed in a computer (22) using an energy value (A) of at least one signal pulse.

**Patentansprüche**

**1.** Verfahren zur Bestimmung der Durchblutung in einem Körperelement (5), wobei das Verfahren die Schritte des Erzeugens elektrischer Messsignale anhand eines nicht-invasiven photometrischen Messvorgangs, des Normalisierens der elektrischen Messsignale und des Bestimmens eines Perfusionsindex (Perf) anhand der normalisierten Signale umfasst, **dadurch gekennzeichnet, dass** der Perfusionsindex anhand der normalisierten Signale bestimmt wird, wobei ein Energiewert (A) von mindestens einem Signalimpuls verwendet wird.

**2.** Verfahren nach Anspruch 1, wobei das Normalisieren der elektrischen Messsignale das Normalisieren der elektrischen Messsignale in Bezug auf den Gleichstrompegel der elektrischen Messsignale umfasst.

**3.** Verfahren nach Anspruch 1, wobei die Signalkurve vom Ende der Diastole eines Herzschlags zum Ende der Diastole des nächsten Herzschlags zur Bestimmung des Energiewertes (A) verwendet wird.

**4.** Verfahren nach Anspruch 1, wobei die Signalkurve analysiert wird und nur ein Teil des Impulses zur Bestimmung des Energiewertes (A) verwendet wird.

**5.** Verfahren nach Anspruch 1, wobei die Fläche des Impulses anhand einer Anzahl von vordefinierten geometrischen Figuren geschätzt wird, um einen Energiewert (A) zu bestimmen.

**6.** Verfahren nach Anspruch 1, wobei die Auswirkung der Blutsättigung auf die Signale für andere Wellenlängen als die isobestische Wellenlänge kompensiert wird.

**7.** Verfahren nach Anspruch 1, wobei die Signale von dem Sensor vorgefiltert und/oder artefaktreduziert werden, bevor die Durchblutung bestimmt wird.

**8.** Vorrichtung (20) zur Bestimmung der Durchblutung in einem Körperelement (5), mit einer Signalkomponente (21), die vorgesehen ist, um elektrische Messsignale anhand eines nicht-invasiven photometrischen Messvorgangs zu erzeugen, und **gekennzeichnet durch** eine Bestimmungskomponente (22), die vorgesehen ist, um die elektrischen Messsignale zu normalisieren und einen Perfusionsindex (Perf) anhand der normalisierten Signale zu bestimmen, wobei ein Energiewert (A) von mindestens einem Signalimpuls verwendet wird.

**9.** Vorrichtung (20) nach Anspruch 8, wobei die Signalkomponente (21) vorgesehen ist, um anhand eines nicht-invasiven photometrischen Messvorgangs elektrische Messsignale zur Bestimmung der arteriellen Sauerstoffsättigung des Blutes zu erzeugen, wobei die Vorrichtung (20) weiterhin Filtermittel (24) umfasst, um die Auswirkung von Signalartefakten zu reduzieren oder zu eliminieren, und wobei die Bestimmungskomponente (22) vorgesehen ist, um den Perfusionsindex (Perf) anhand der vorgefilterten und artefaktreduzierten Signale zu bestimmen.

**10.** Computerprogramm (23) zur Bestimmung der Durchblutung in einem Körperelement (5), mit Computerprogrammanweisungen zum Bestimmen eines Perfusionsindex (Perf) anhand normalisierter elektrischer Messsignale aus einem nicht-invasiven photometrischen Messvorgang, wenn das Computerprogramm (23) in einem Computer (22) ausgeführt wird, wobei ein Energiewert (A) von mindestens einem Signalimpuls verwendet wird.

## Revendications

**1.** Procédé pour déterminer la perfusion de sang dans un membre du corps (5), le procédé comprenant les étapes consistant à produire des signaux de mesure électriques à partir d'un processus de mesure photométrique non invasif, normaliser les signaux de mesure électriques et déterminer un index de perfusion (Perf) à partir des signaux normalisés, **caractérisé en ce que** l'index de perfusion est déterminé à partir des signaux normalisés, en utilisant une valeur énergétique (A) d'au moins une impulsion de signal.

**2.** Procédé selon la revendication 1, dans lequel la normalisation des signaux de mesure électriques consiste à normaliser les signaux de mesure électriques par rapport au niveau de courant direct des signaux de mesure électriques.

**3.** Procédé selon la revendication 1, dans lequel la courbe du signal de la fin de la diastole d'un battement cardiaque à la fin de la diastole du battement cardiaque suivant est utilisée pour déterminer la valeur énergétique (A).

**4.** Procédé selon la revendication 1, dans lequel la courbe de signal est analysée et seule une partie de l'impulsion est utilisée pour déterminer la valeur énergétique (A).

**5.** Procédé selon la revendication 1, dans lequel la zone de l'impulsion est estimée en utilisant un nombre de figures géométriques prédéfinies afin de déterminer une valeur énergétique (A).

**6.** Procédé selon la revendication 1, dans lequel l'impact de la saturation sanguine sur les signaux pour des longueurs d'onde autres que la longueur d'onde isobestique est compensé.

**7.** Procédé selon la revendication 1, dans lequel les signaux provenant du capteur sont pré-filtrés et/ou réduits en artéfact avant la détermination de la perfusion.

**8.** Dispositif (20) pour déterminer la perfusion du sang dans un membre du corps (5), comprenant un composant de signal (21) adapté pour produire des signaux de mesure électriques à partir d'un processus de mesure photométrique non invasif, et **caractérisé par** un composant de détermination (22) conçu pour normaliser les signaux de mesure électriques et pour déterminer un index de perfusion (Perf) à partir des signaux normalisés, en utilisant une valeur énergétique (A) d'au moins une impulsion de signal.

**9.** Dispositif (20) selon la revendication 8, dans lequel le composant de signal (21) est conçu pour produire des signaux de mesure électriques à partir d'un processus de mesure photométrique non invasif pour déterminer la saturation du sang artériel en oxygène, le dispositif (20) comprenant en outre des moyens de filtrage (24) pour réduire ou éliminer l'effet des artéfacts de signal, et dans lequel le composant de détermination (22) est conçu pour déterminer l'index de perfusion (Perf) à partir des signaux pré-filtrés et réduits en artéfact.

10. Programme informatique (23) pour déterminer la perfusion du sang dans un membre de corps (5), comprenant des instructions de programme informatique pour déterminer un index de perfusion (Perf) à partir de signaux de mesure électriques normalisés issus d'un processus de mesure photométrique non invasif, lorsque le programme informatique (23) est exécuté dans un ordinateur (22), **caractérisé en ce que** l'index de perfusion est déterminé à partir des signaux normalisés en utilisant une valeur énergétique (A) d'au moins une impulsion de signal.

FIG. 1

FIG. 2

Photodiode
intensity

I

$i_2(t)$

$I_2(t)$

12

$i_1(t)$

$I_1(t)$

11

Imax=
$Idc(\lambda_2)$

Imin($\lambda_2$)

$Idc(\lambda 1)$

t

## FIG. 3

Intensitity attenuation (absolute)

$i_1(t)$

13

0

t

$i_2(t)$

14

0

t

## FIG. 4

M

FIG. 5

M

FIG. 6

M

FIG. 7

M

FIG. 8

## FIG. 9

## FIG. 10

Light Absorption for Hemoglobin and Oxyhemoglobin

## FIG. 11

FIG. 12

FIG. 13

FIG. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4867165 A
- EP 0807402 A